# FASCICULE DE BREVET EUROPEEN

(11) **EP 3 666 880 B1**
(45) Date de publication et mention de la délivrance du brevet: **14.04.2021**
(21) Numéro de dépôt: 19216684.1
(22) Date de dépôt: 16.12.2019
(51) Int. Cl.: C12M 1/107, C10L 3/08, C12M 1/00

(54) **PROCÉDÉ COUPLÉ DE MÉTHANISATION ET DE MÉTHANATION**
GEKOPPELTES METHANISIERUNGS- UND BIOGASVERFAHREN
COMBINED METHOD FOR ANAEROBIC DIGESTION AND METHANATION

(30) Priorité: 14.12.2018 FR 1872930
(43) Date de publication de la demande: 17.06.2020
(73) Titulaire: Storengy, 92270 Bois-Colombes (FR)
(72) Inventeur: NGAMENI JIEMBOU, Joseph Rostand, 91260 JUVISY (FR)
(74) Mandataire: Cornuejols, Georges

(56) Documents cités:
- EP-A1- 2 982 740
- EP-A2- 1 574 581
- WO-A1-2015/155427
- DE-A1-102011 103 430
- US-A1- 2015 284 651

## Description

### DOMAINE TECHNIQUE DE L'INVENTION

La présente invention concerne le domaine de la production industrielle de méthane.

La présente invention concerne en particulier un procédé combinant un circuit de production de méthane à partir de biogaz et un circuit de production de méthane de synthèse par une unité de méthanation.

La présente invention trouve une application particulièrement avantageuse dans la mise en œuvre d'un système de type power-to-gas permettant de transformer l'électricité, notamment les excédents électriques issus de sources renouvelables et les excédents électriques à bas coût, en gaz hydrogène utilisé pour alimenter une unité de méthanation.

### ÉTAT DE LA TECHNIQUE

Les systèmes power-to-gas (pour « de la puissance électrique au gaz », aussi abrégé « P2G ») permettent de transformer l'électricité, notamment l'électricité issue d'énergies renouvelables, en gaz hydrogène qui pourra servir de matière première pour de la méthanation. Ainsi converties, ces énergies peuvent être stockées et transportées dans les réseaux de gaz.

On distingue la méthanation de synthèse par voie catalytique, qui est la seule concernée par l'invention, de la méthanation biologique. La méthanation biologique, notamment illustrée par les installations décrites dans les documents EP 1 574 581 A2 et EP 2 982 740 A1, consiste à produire du méthane par hydrogénation du CO₂ à partir de populations microbiennes.

Les systèmes power-to-gas reposant sur une méthanation de synthèse requièrent deux éléments principaux, un électrolyseur pour la production d'hydrogène à partir d'électricité et d'eau, et une unité de méthanation pour la conversion d'hydrogène, « H₂ », et de dioxyde de carbone, « CO₂ », en méthane de synthèse, en présence d'un catalyseur.

Pour faire fonctionner une unité de méthanation de synthèse, il est nécessaire de disposer d'une source de CO₂, cette source peut être industrielle c'est-à-dire provenir de fumées ou d'épurateurs d'air. Le CO₂ peut provenir également d'une unité de gazéification (syngas) ou d'une unité de méthanisation (biogaz).

En outre ces systèmes comportent un ensemble d'équipements nécessaires à leur intégration et à la mise aux spécifications du gaz pour injection dans les infrastructures du gaz naturel.

En effet, pour un taux de conversion de CO₂ de l'ordre de 90%, le mélange de gaz en sortie d'un système power-to-gas comporte une composition en volume de H₂ trop grande, de l'ordre de 29%, et une composition en volume de CO₂ trop grande, de l'ordre de 7%. Afin de parvenir à une mise en conformité du méthane de synthèse issu du système power-to-gas aux spécifications des réseaux, plusieurs solutions sont envisagées dans l'art antérieur.

Une première solution consiste à utiliser une unité de séparation de gaz pour la mise aux spécifications du méthane de synthèse distincte de celle utilisée pour la mise aux spécifications du biométhane. Cette solution ne permet pas de valoriser les gaz non convertis (H₂ et CO₂) au sein de l'unité de méthanation qui représentent environ 10% des réactifs et environ 36 % du gaz en sortie de système power-to-gas.

Une deuxième solution consiste à alimenter le réacteur de méthanation directement par un flux de biogaz et à utiliser une seule unité de séparation de gaz, placée en aval du système power-to-gas, pour une mise aux spécifications. Dans cette solution comme dans la précédente les gaz non convertis ne sont pas valorisés et représentent une perte non négligeable. Cette solution présente également le défaut qu'elle suppose un fonctionnement à haute pression pour permettre la dilution du CO₂ dans l'unité de méthanation. Cette solution présente également le défaut que la présence du biométhane en quantité importante dans l'unité de méthanation conduit à des dépôts de carbone susceptibles de l'endommager.

Une troisième solution consiste à alimenter le réacteur de méthanation directement par un flux de biogaz et à mettre en œuvre un séparateur de gaz afin de recycler une partie des gaz non convertis en sortie du réacteur de méthanation. Une solution de ce type est par exemple illustrée par l'installation décrite dans le document US 2015/0284651. Cette solution permet de valoriser les gaz non convertis mais les problèmes de dilution de CO₂ et de dépôt de carbone dû à une alimentation du réacteur de méthanation en biogaz et non en CO₂ pur demeurent. L'hydrogène étant présent dans les gaz non convertis, ce gaz pourrait modifier les conditions de fonctionnement et performances de certains équipements du circuit de production de méthane à partir de biogaz, notamment les compresseurs, prévus pour fonctionner uniquement en présence du biogaz.

Aucunes des solutions actuelles combinant méthanisation et méthanation de synthèse ne permettent de répondre à tous les besoins requis, à savoir de limiter les pertes économiques dues aux gaz non convertis, H₂ et CO₂, et de prévenir les dégâts causés par ces gaz aux installations. Tout particulièrement, aucune solution existante ne permet l'adaptation d'une installation de méthanisation pour fournir l'intrant CO₂ nécessaire au fonctionnement d'une installation de type power-to-gas par méthanation de synthèse par voie catalytique.

### EXPOSE DE L'INVENTION

La présente invention a pour objectif de répondre au moins en partie aux limitations de l'art antérieur précédemment évoqué.

À cet effet, selon un premier aspect, la présente invention vise un procédé de production de méthane, comportant :
- une étape de production de biogaz par un digesteur,
- une étape de séparation d'un flux de dioxyde de carbone d'un flux alimenté au moins en partie par le flux de biogaz issu de l'étape de production de biogaz,
- une étape de fourniture du flux de dioxyde de carbone à un réacteur de méthanation,
- une étape de production de gaz de synthèse par un réacteur de méthanation au moins à partir du flux de dioxyde de carbone fourni au réacteur et d'un flux riche en dihydrogène,
- une étape de séparation du gaz de synthèse en une fraction riche en dihydrogène d'une part et une fraction riche en méthane et en dioxyde de carbone d'autre part et
- une étape de recirculation de la fraction riche en dihydrogène pour fournir ladite fraction au réacteur de méthanation.

Grâce à ces dispositions, le procédé objet de l'invention permet de récupérer le CO₂ contenu dans le biogaz issu du digesteur et d'alimenter une unité de méthanation du système power-to-gas en CO₂. Le procédé objet de l'invention permet de faire recirculer les gaz non convertis H₂ et CO₂ au sein de l'unité de méthanation et ainsi de limiter les pertes économiques dues aux non-valorisations de ces gaz.

En outre, le procédé objet de l'invention permet d'éviter la circulation du méthane, contenu en quantité importante dans le biogaz, dans l'unité de méthanation. Cette caractéristique permet de prévenir les dépôts de carbone susceptibles de réduire la surface de contact entre le catalyseur du réacteur de méthanation de synthèse et le gaz circulant et ainsi de prévenir la baisse de rendement du réacteur de méthanation de synthèse.

Enfin, ce procédé permet de faire fonctionner les équipements nécessaires à l'intégration de l'unité de méthanisation et du système power-to-gas sans modifier leurs conditions opératoires ou en maintenant les conditions opératoires similaires lorsque ces systèmes fonctionnent indépendamment l'un de l'autre. En particulier, la séparation du H₂ dans un séparateur dédié et sa recirculation vers l'unité de méthanation permet d'empêcher sa circulation dans des conduites non adaptées et ainsi de se prémunir des risques de fragilisation de ces conduites. Ce risque émerge dans le cadre de l'adaptation d'une installation de méthanisation pour fournir l'intrant CO₂ nécessaire au fonctionnement d'une installation de type power-to-gas. Dans ce cas de figure, du gaz comportant de l'hydrogène est amené à circuler dans des conduites de gaz et des équipements non prévus pour faire circuler un gaz comportant de l'hydrogène.

Dans des modes de réalisation, le procédé de production de méthane comporte:
- une étape de production de dihydrogène par électrolyse, et
- une étape de fourniture du dihydrogène produit au réacteur de méthanation.

Grâce à ces dispositions, l'énergie électrique peut être utilisée, en combinaison avec un apport d'eau, pour produire l'hydrogène nécessaire à la méthanation. De manière avantageuse, l'énergie électrique provient de sources intermittentes telles que l'énergie solaire ou éolienne ou est utilisée seulement lorsque des excédents sont constatés sur le réseau électrique.

Dans des modes de réalisation, le procédé de production de méthane comporte :
- une étape de mélange du biogaz avec la fraction riche en méthane et en dioxyde de carbone issu de la méthanation,
- une étape de séparation du mélange obtenu en une fraction riche en méthane et une fraction riche en dioxyde de carbone.

Grâce à ces dispositions, le procédé objet de l'invention permet de mettre concomitamment aux spécifications des réseaux de gaz naturel le biométhane issu de la méthanisation réalisée par le digesteur et le méthane de synthèse produit par le système power-to-gas.

Dans des modes de réalisation, la fraction riche en méthane a une teneur en hydrogène conforme aux spécifications des réseaux de gaz naturel.

Par exemple, la fraction riche en méthane a une teneur en hydrogène inférieure à 2,5 pourcents molaires et une teneur en dioxyde de carbone inférieure à 6 pourcents molaires.

Grâce à ces dispositions, cette fraction riche en méthane est mise aux spécifications et peut être utilisée pour alimenter un réseau de gaz naturel.

Dans des modes de réalisation, le procédé de production de méthane comporte, en amont de l'étape de mélange :
- une étape de désulfuration du biogaz produit par le digesteur, et
- une étape de déshydratation du biogaz produit par le digesteur.

On précise que l'étape de désulfuration peut être réalisée après l'étape de déshydratation du biogaz.

Dans des modes de réalisation, le procédé de production de méthane comporte une étape de mélange du dihydrogène produit par électrolyse avec la fraction riche en dihydrogène issu de l'étape de séparation du gaz de synthèse.

Grâce à ces dispositions, le réacteur de méthanation est alimenté en dihydrogène par un flux unique de dihydrogène nouvellement formé et de dihydrogène recirculé.

Dans des modes de réalisation, le procédé de production de méthane comporte, en amont de l'étape de séparation du gaz de synthèse, une étape de déshydratation du gaz de synthèse produit par le réacteur de méthanation.

Dans des modes de réalisation, l'unité de méthanation est alimentée par un flux de gaz comportant un mélange stœchiométrique de CO₂ et de H₂.

Grâce à ces dispositions, le rendement énergétique global du procédé de production de méthane est amélioré. En particulier, le rendement énergétique est meilleur par rapport au rendement d'un réacteur de méthanation alimenté par un mélange surstœchiométrique en hydrogène. La fourniture d'un mélange surstœchiométrique en hydrogène permet d'obtenir un meilleur taux de conversion du CO₂ en méthane mais un tel mélange n'est pas nécessaire dans le cadre de certains modes de réalisation de l'invention, lorsque le CO₂ non converti par le réacteur de méthanation est récupéré puis valorisé.

Selon un deuxième aspect, l'invention vise un dispositif d'adaptation d'une unité de production de méthane à partir de biogaz en une installation de production de méthane power-to-gas, qui comporte :
- un réacteur de méthanation configuré pour produire un gaz de synthèse au moins à partir du flux de dioxyde de carbone fourni au réacteur et d'un flux riche en dihydrogène,
- un moyen de fourniture au réacteur de méthanation d'un flux de dioxyde de carbone issu au moins en partie d'un moyen de séparation du dioxyde de carbone d'un flux comportant du biogaz produit par un digesteur,
- un séparateur du gaz de synthèse en une fraction riche en dihydrogène d'une part et une fraction riche en méthane et en dioxyde de carbone d'autre part,
- une conduite de recirculation de la fraction riche en dihydrogène pour fournir ladite fraction au réacteur de méthanation, et
- une conduite de raccordement pour fournir la fraction riche en méthane et en dioxyde de carbone au moyen de séparation du dioxyde de carbone.

Grâce à ces dispositions, une installation existante de production de biométhane comportant un digesteur, des moyens de désulfuration et de déshydratation et une unité de séparation du CO₂ peut être adaptée en une installation combinée de méthanation de synthèse et de méthanisation permettant ainsi la valorisation du CO₂ extrait du biogaz.

Cette solution est particulièrement avantageuse, car elle ne nécessite pas de modification majeure de l'installation existante de biométhane ni de modification majeure de son fonctionnement. Les conditions de fonctionnement et performances des équipements de l'installation de production de biométhane, notamment les compresseurs, idéalement prévus pour fonctionner uniquement en présence du biogaz, ne sont pas modifiés.

En d'autres termes, dans le cadre de l'adaptation d'une installation de méthanisation pour fournir l'intrant CO₂ nécessaire au fonctionnement d'une installation de type power-to-gas, on s'affranchit du risque crée par la fourniture d'un gaz riche en H₂ aux conduites de gaz et aux équipements de l'installation de méthanisation. Ce risque est celui de la fragilisation des conduites de gaz et celui de dégâts causés aux équipements par le gaz riche en H₂.

Les avantages, buts et caractéristiques du dispositif d'adaptation d'une installation de production de méthane objet de l'invention, étant similaires à ceux du procédé objet de l'invention, ils ne sont pas rappelés ici.

Selon un troisième aspect, l'invention vise une installation de production de méthane, qui comporte :
- un digesteur configuré pour produire du biogaz,
- un séparateur d'un flux de dioxyde de carbone d'un flux composé au moins en partie d'un flux de biogaz issu du digesteur,
- un moyen de fourniture du flux de dioxyde de carbone à un réacteur de méthanation,
- un réacteur de méthanation configuré pour produire un gaz de synthèse au moins à partir du flux riche en dioxyde de carbone fourni au réacteur et d'un flux riche en dihydrogène,
- un séparateur du gaz de synthèse en une fraction riche en dihydrogène d'une part et une fraction riche en méthane et en dioxyde de carbone d'autre part, et
- une conduite de recirculation de la fraction riche en dihydrogène pour fournir le réacteur de méthanation en dihydrogène.

Dans des modes de réalisation, l'installation comporte un mixeur configuré pour mélanger le biogaz avec la fraction riche en méthane et en dioxyde de carbone issu de la méthanation, ledit mixeur étant placé en amont du séparateur configuré pour séparer le mélange obtenu en une fraction riche en méthane et une fraction riche en dioxyde de carbone.

Dans des modes de réalisation, l'installation comporte un électrolyseur pour la production d'hydrogène pour fournir le réacteur de méthanation.

Dans des modes de réalisation, l'installation comporte une unité de désulfuration du biogaz, une unité de déshydratation du biogaz et une unité de déshydratation du gaz de synthèse.

Dans des modes de réalisation, l'installation comporte un mixeur configuré pour mélanger la fraction riche en dihydrogène acheminée depuis le séparateur d'hydrogène avec le dihydrogène produit par l'électrolyseur en vue d'alimenter le réacteur de méthanation.

Les avantages, buts et caractéristiques de l'installation objet de l'invention, étant similaires à ceux du procédé objet de l'invention, ils ne sont pas rappelés ici.

### BRÈVE DESCRIPTION DES FIGURES

D'autres avantages, buts et caractéristiques particulières de l'invention ressortiront de la description non limitative qui suit d'au moins un mode de réalisation particulier du dispositif et du procédé objets de la présente invention, en regard des dessins annexés, dans lesquels :
[Fig 1] La figure 1 représente, sous forme de schéma, un mode de réalisation particulier de l'installation objet de l'invention et
[Fig 2] La figure 2 représente, sous forme de logigramme, un mode de réalisation particulier du procédé objet de l'invention.

### DESCRIPTION D'EXEMPLES DE RÉALISATION DE L'INVENTION

La présente description est donnée à titre non limitatif, chaque caractéristique d'un mode de réalisation pouvant être combinée à toute autre caractéristique de tout autre mode de réalisation de manière avantageuse.

On note, dès à présent, que les figures ne sont pas à l'échelle.

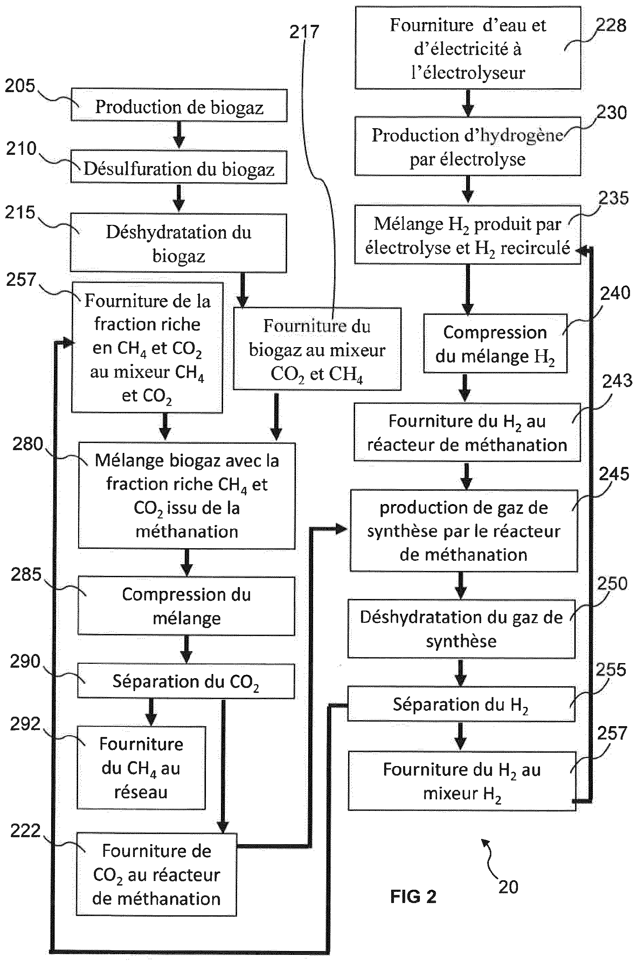

On observe en figure 2, un procédé 20 de production de méthane CH₄ par la mise en œuvre couplée de méthanisation et de méthanation.

La méthanisation est la production de méthane par voie biologique à partir de biogaz issu de la digestion de biomasse. La méthanisation est aussi appelée digestion anaérobie. On désigne souvent par le terme biométhane le méthane obtenu par de cette manière.

Dans la présente rédaction, le terme de méthanation désigne la méthanation par voie catalytique. La méthanation par voie catalytique est la réaction de synthèse du méthane à partir de dihydrogène H₂ et de monoxyde de carbone CO ou de dioxyde de carbone CO₂ en présence d'un catalyseur. On désigne par les termes méthane de synthèse le méthane obtenu de cette manière.

La mise en oeuvre couplée des procédés de méthanisation et de méthanation présente l'avantage de valoriser au moins en partie le CO₂ contenu dans le biogaz en fournissant ce CO₂ comme matière première utile à la production de méthane de synthèse par méthanation.

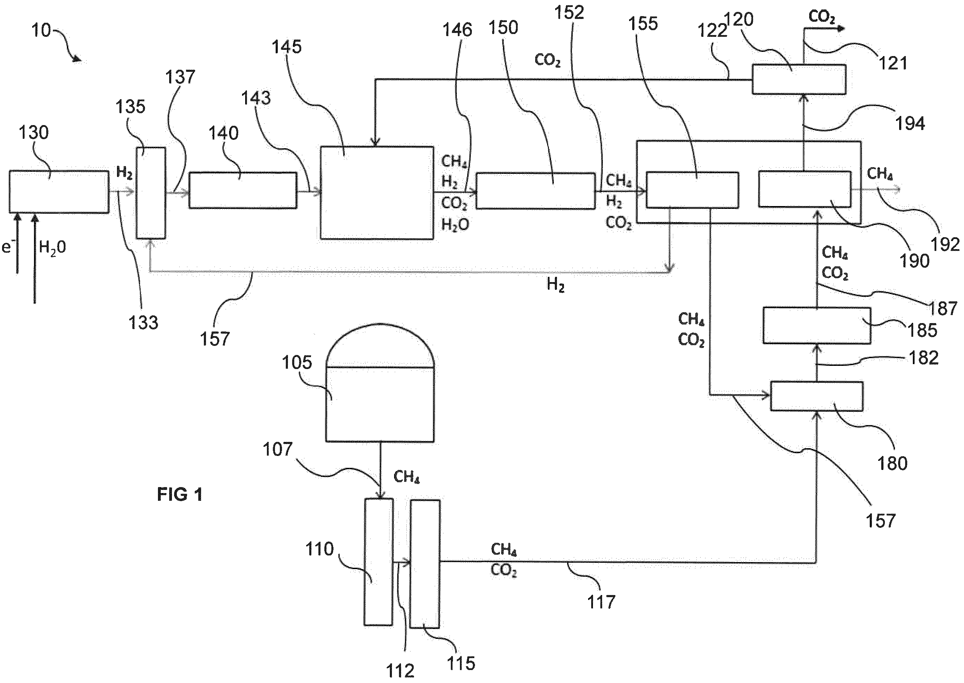

Le procédé objet de l'invention sera mieux compris au regard du schéma d'installation 10 présenté en figure 1 qui illustre un mode de réalisation particulier d'une installation de production de méthane objet de l'invention.

Le procédé de production de méthane 20 comporte une étape 205 de production de biogaz par un digesteur 105. Le digesteur peut également être appelé méthaniseur. Le biogaz produit est appelé biogaz brut. Le biogaz brut comporte du méthane, du dioxyde de carbone, de la vapeur d'eau, du soufre et d'autres contaminants. Dans des modes de réalisation, le biogaz brut est composé au moins à 50% de méthane.

Le biogaz brut produit est acheminé par une conduite de gaz 107 vers une unité 110 de désulfuration.

Dans des modes de réalisation, lors d'une étape 210 de désulfuration, une partie du soufre est séparée du biogaz brut. Le biogaz brut désulfuré est fourni par une conduite de gaz 112 à une unité de déshydratation.

Dans des modes de réalisation, lors d'une étape 215 de déshydratation, une partie de la vapeur d'eau est séparée du biogaz brut désulfuré.

On précise que les étapes 210 de désulfuration et 215 de déshydratation peuvent être interverties.

Lors d'une étape 217, le biogaz obtenu est fourni par une conduite 117 à un mixeur 180. À titre d'exemple, le biogaz acheminé par la conduite 117 a une composition de 55% CH₄ et 45% CO₂.

En outre, le mixeur 180 est relié à une conduite 157. La conduite 157 fournit le mixeur 180 en une fraction riche en méthane et en dioxyde de carbone issu du circuit de méthanation. Le circuit de méthanation sera détaillé par la suite.

Dans des modes de réalisation, lors d'une étape 280 de mélange du biogaz avec la fraction riche en méthane et en dioxyde de carbone issu de la méthanation les deux flux sont mélangés par le mixeur 180. Cette étape permet d'assurer que les gaz issus des deux flux sont à la même pression avant aspiration dans le compresseur 185 à CH₄ et CO₂. Le mélange résultant de l'étape 280 est fourni à un compresseur 185 à CH₄ et CO₂ par une conduite 182.

Dans des modes de réalisation, lors d'une étape 285 de compression du mélange, le compresseur 185 comprime le mélange. Cette étape 285 a pour effet de compenser les pertes de charges en sortie de circuit, au niveau de la conduite 192. À l'issue de l'étape 285 le mélange est fourni à un séparateur de CO₂ 190 via une conduite 187.

Le compresseur 185 a pour rôle de fournir un gaz à la pression du réseau.

Lors d'une étape 290 de séparation du CO₂ le mélange de gaz est séparé en une fraction riche en méthane et une fraction riche en CO₂ par le séparateur de CO₂ 190.

La séparation du CO₂ peut être réalisée par toute méthode connue de l'homme du métier, par exemple par perméation membranaire, par adsorption aussi appelée PSA (abrévié de l'anglais : *Pressure Swing Adsorption),* par absorption, par lavage à l'eau ou par lavage aux solvants, par lavage aux amines ou encore par cristallisation ou liquéfaction du CO₂.

De manière préférentielle, la séparation du CO₂ est réalisée par une méthode de perméation membranaire.

Dans des modes de réalisation, la fraction riche en méthane a une teneur en hydrogène inférieure à 2,5 pourcents molaires et une teneur en dioxyde de carbone inférieure à 6 pourcents molaires. Dans ces conditions, le méthane respecte les spécifications de réseau et pourra être fourni directement au réseau de gaz naturel au cours d'une étape 292.

La fraction riche en CO₂ en sortie du séparateur de CO₂ 190 est acheminée par la conduite 194 vers le splitter 120. Le splitter 120 sépare le flux de CO₂ issu de la conduite 194 en un premier flux de CO₂ et un second flux de CO₂. Le premier flux de CO₂ est acheminé lors d'une étape 222, par la conduite 122, en vue de fournir à l'unité de méthanation la quantité de CO₂ requise pour son fonctionnement. Le second flux de CO₂ est évacué de l'installation par la conduite 121 en vue d'être stocké, valorisé dans le cadre d'un autre processus ou relâché dans l'atmosphère.

Dans un mode de réalisation préférentiel, le splitter 120 dimensionne le premier flux de CO₂ de sorte à former un mélange stœchiométrique de CO₂ et de H₂ dans l'unité de méthanation.

Dans un mode de réalisation préférentiel, le splitter 120 sépare le flux de CO₂ issu de la conduite 194 en un premier flux de CO₂ et un second flux de CO₂ seulement lorsqu'un excès de CO₂ est détecté dans la fraction riche en CO₂ en sortie du séparateur de CO₂ 190.

L'unité de méthanation 145 est alimentée d'une part en CO₂ par la conduite 122 et d'autre par en H₂ par la conduite 143. La fourniture en H₂ de l'unité de méthanisation est décrite ci-dessous.

Lors d'une étape 228 de fourniture en eau et en électricité à l'électrolyseur 130, ces éléments nécessaires au fonctionnement de l'électrolyseur lui sont apportés.

Dans un mode de réalisation avantageux, l'électricité fournie à l'électrolyseur provient d'une grille alimentée au moins en partie par des sources d'énergie intermittente telles que l'énergie solaire ou éolienne ou est utilisée seulement lorsque des excédents sont disponibles sur le réseau électrique.

Lors d'une étape 230, l'électrolyseur 130 produit l'hydrogène nécessaire au fonctionnement de l'unité de méthanation 145. L'hydrogène produit est fourni au mixeur 135 par la conduite de gaz 133.

Lors d'une étape 235 l'hydrogène produit lors de l'étape 230 est mélangé à de l'hydrogène recirculé. La séparation et la recirculation de l'hydrogène non convertis lors de la méthanation interviennent aux étapes 255 et 257 qui seront décrites par la suite. Le flux d'hydrogène mélangé est fourni par la conduite 137 au compresseur 140.

Dans des modes de réalisation, une étape 240 de compression est mise en œuvre. Lors de l'étape 240 de compression du mélange d'hydrogène, le mélange est comprimé par le compresseur 140 jusqu'à atteindre la pression de fonctionnement du réacteur de méthanation 145. Le mélange comprimé est fourni par une conduite 143 au réacteur de méthanation 145.

Dans des modes de réalisation, le mélange d'hydrogène est comprimé à une pression fixée entre 1 et 100 bars, par exemple 30 bars.

Dans des modes de réalisation, l'électrolyseur produit un flux d'hydrogène déjà sous pression entre 20 et 100 bars, l'étape de compression n'est alors pas nécessaire.

Comme décrit ci-dessus le réacteur de méthanation 145 est alimenté en dioxyde de carbone provenant de la conduite 122, d'une part, et par un flux riche en dihydrogène provenant de la conduite 143, d'autre part.

Lors d'une étape 245 de production le réacteur de méthanation 145 produit du gaz de synthèse à partir du flux de dioxyde de carbone et du flux riche en dihydrogène.

Dans des modes de réalisation, le réacteur de méthanation 145 est alimenté par un flux de gaz comportant un mélange stœchiométrique de CO₂ et de H₂.

Le gaz de synthèse produit lors de l'étape 245 comporte du méthane, de la vapeur d'eau et des gaz non convertis : le dihydrogène et le dioxyde de carbone. Le gaz de synthèse produit lors de l'étape 245 est fourni via une conduite 146 à l'unité de déshydratation 150.

Dans des modes de réalisation, lors d'une étape 250 le gaz de synthèse est déshydraté par une unité de déshydratation 150. Le gaz de synthèse déshydraté est fourni à un séparateur de dihydrogène 155 via une conduite de gaz 152.

Lors d'une étape 255 le gaz de synthèse est séparé par le séparateur de dihydrogène 155 en une fraction riche en dihydrogène d'une part et une fraction riche en méthane et en dioxyde de carbone d'autre part.

Lors d'une étape 257 la fraction riche en dihydrogène est recirculée par la conduite 157 vers le mixeur 135 en vue d'alimenter le réacteur de méthanation 145. Ces dispositions avantageuses permettent de se prémunir de la perte de l'hydrogène non converti lors de l'étape de méthanation. De plus, ces dispositions avantageuses permettent de se prémunir du risque de fournir au circuit méthanisation un gaz riche en dihydrogène susceptible d'endommager les équipements et conduites dudit circuit.

Lors d'une étape 257 la fraction riche en méthane et en dioxyde de carbone est fournie au mixeur 180 via une conduite de gaz 157.

Comme décrit plus haut, lors d'une étape 280, la fraction riche en méthane et en dioxyde de carbone issu de la méthanation est mélangée par le mixeur 180 avec le biogaz désulfuré et déshydraté issu du digesteur 105.

L'étape 290 de séparation du CO₂, décrite plus haut, est réalisée sur le mélange de gaz composé par la fraction riche en méthane et en dioxyde de carbone issu de la méthanation et par le biogaz désulfuré et déshydraté issu de la méthanisation. Ces dispositions avantageuses permettent de se prémunir de la perte du dioxyde de carbone non converti lors de l'étape de méthanation.

Dans des modes de réalisation, le circuit de production de méthane à partir de biogaz comportant le digesteur 104, le compresseur 185 et le séparateur de CO₂ 190 peut fonctionner indépendamment de la mise en route du circuit de production de méthane à partir de gaz de synthèse par le réacteur de méthanation 145.

À titre d'exemple, on pourra faire fonctionner l'unité de production de méthane à partir de biogaz en continu et faire fonctionner le circuit de production de méthane à partir de gaz de synthèse par le réacteur de méthanation 145 de manière intermittente.

Dans ce cas de figure, lorsque le fonctionnement du circuit de production de méthane à partir de gaz de synthèse est stoppé, les dispositions suivantes sont mises en œuvre :
- les équipements de l'unité de production de méthane à partir de biogaz fonctionnent en continu,
- la conduite de gaz 157 est fermée,
- la conduite de gaz 194 est fermée, et
- les équipements 120, 130, 135, 140, 145, 150, et 155 sont stoppés.

De manière particulièrement avantageuse, le circuit de production de méthane à partir de gaz de synthèse est mis en route lorsque des excédents d'énergie électrique sont disponibles ou que l'électricité est bon marché et au contraire arrêté lorsqu'aucun excédent d'électricité n'est disponible ou lorsque l'électricité est chère.

Ci-après, on décrit un mode particulier de réalisation de l'invention se rapportant à un dispositif d'adaptation d'une installation de méthanisation pour fournir l'intrant CO₂ nécessaire au fonctionnement d'une installation de type power-to-gas.

Ce mode de réalisation vise à convertir une unité de production de méthane à partir de biogaz déjà en place en une installation de production de méthane power-to-gas du type de celle illustrée en figure 1. Ladite installation combinant une production de méthane par méthanisation et par méthanation, à partir de biogaz et de gaz de synthèse.

En référence à la figure 1, on précise qu'une unité de production de méthane à partir de biogaz déjà en place pourra par exemple comporter un digesteur 105, une unité de désulfuration 110, une unité de déshydratation 115, un compresseur 185 et un séparateur de CO₂ 190, ainsi que les conduites de gaz 107, 112, 117, 187 et 192 reliant ces différents éléments.

Dans un mode de réalisation, le dispositif d'adaptation d'une installation de méthanisation pour fournir l'intrant CO₂ nécessaire au fonctionnement d'une installation de type power-to-gas comporte :
- un moyen de fourniture au réacteur de méthanation d'un flux de dioxyde de carbone issu au moins en partie du moyen de séparation du dioxyde de carbone 190 d'un flux comportant du biogaz produit par un digesteur,
- un réacteur de méthanation 145 configuré pour produire un gaz de synthèse au moins à partir du flux de dioxyde de carbone fourni au réacteur et d'un flux riche en dihydrogène,
- un séparateur 155 du gaz de synthèse en une fraction riche en dihydrogène d'une part et une fraction riche en méthane et en dioxyde de carbone d'autre part,
- une conduite de gaz 157 de recirculation de la fraction riche en dihydrogène pour fournir ladite fraction au réacteur de méthanation, et
- une conduite de raccordement 157 pour fournir la fraction riche en méthane et en dioxyde de carbone au moyen de séparation 190 du dioxyde de carbone.

Dans des modes de réalisation, le moyen de fourniture au réacteur de méthanation d'un flux de dioxyde de carbone comporte une conduite 194 reliant le séparateur de dioxyde de carbone à un splitter CO₂ 120 et une conduite 122 reliant le splitter CO₂ au réacteur de méthanation.

Dans des modes de réalisation, le dispositif d'adaptation comporte un mixeur 180 visant à mélanger la fraction riche en méthane et en dioxyde de carbone issu de la méthanation avec le biogaz désulfuré et déshydraté issu du digesteur 105. Dans ces modes de réalisation, la conduite de raccordement 157 est reliée au mixeur 105, le mixeur 105 est à son tour relié par une conduite 182 au compresseur 185.

Dans des modes de réalisation, le dispositif d'adaptation comporte une conduite de contournement qui relie directement la conduite 117 à la conduite 182 de sorte à mettre en place une dérivation qui permet au biogaz de contourner le mélangeur uniquement lorsque le circuit de production de méthane à partir de gaz de synthèse n'est pas en fonctionnement.

L'adaptation d'une installation de méthanisation pour fournir l'intrant CO₂ nécessaire au fonctionnement d'une installation de type power-to-gas au moyen du dispositif d'adaptation objet de l'invention est particulièrement avantageuse. En effet, cette adaptation ne nécessite pas de modification des conduites de gaz 117, 182, 187 et 192 ni d'adaptation importante des équipements en place et en particulier du séparateur de CO₂ 190. En effet ces conduites et équipements risquent d'être endommagés par la circulation d'un gaz comportant une proportion importante de dihydrogène, dans le cadre de l'invention le dihydrogène est séparé en amont par le séparateur 155.

## Revendications

1. Procédé (20) de production de méthane, comportant :
- une étape (205) de production de biogaz par un digesteur,
- une étape (290) de séparation d'un flux de dioxyde de carbone d'un flux alimenté au moins en partie par le flux de biogaz issu de l'étape de production de biogaz,
- une étape (222) de fourniture du flux de dioxyde de carbone à un réacteur de méthanation,
- une étape (245) de production de gaz de synthèse par un réacteur de méthanation au moins à partir du flux de dioxyde de carbone fourni au réacteur et d'un flux riche en dihydrogène,
- une étape (255) de séparation du gaz de synthèse en une fraction riche en dihydrogène d'une part et une fraction riche en méthane et en dioxyde de carbone d'autre part et
- une étape (257) de recirculation de la fraction riche en dihydrogène pour fournir ladite fraction au réacteur de méthanation.

2. Procédé (20) de production de méthane selon la revendication 1, qui comporte :
- une étape (280) de mélange du biogaz avec la fraction riche en méthane et en dioxyde de carbone issu de la méthanation,
- une étape (290) de séparation du mélange obtenu en une fraction riche en méthane et une fraction riche en dioxyde de carbone.

3. Procédé (20) de production de méthane selon la revendication 2, qui comporte, en amont de l'étape (280) de mélange :
- une étape (210) de désulfuration du biogaz produit par le digesteur, et
- une étape (215) de déshydratation du biogaz produit par le digesteur.

4. Procédé (20) de production de méthane selon l'une des revendications 1 à 3, dans lequel la fraction riche en méthane a une teneur en hydrogène conforme aux spécifications des réseaux de gaz naturel.

5. Procédé (20) de production de méthane selon l'une des revendications 1 à 4, qui comporte :
- une étape (230) de production de dihydrogène par électrolyse, et
- une étape (243) de fourniture du dihydrogène produit au réacteur de méthanation.

6. Procédé (20) de production de méthane selon l'une des revendications 1 à 5, qui comporte une étape (235) de mélange du dihydrogène produit par électrolyse avec la fraction riche en dihydrogène issu de l'étape (255) de séparation du gaz de synthèse.

7. Procédé (20) de production de méthane selon l'une des revendications 1 à 6, qui comporte, en amont de l'étape (255) de séparation du gaz, une étape (250) de déshydratation du gaz de synthèse produit par le réacteur de méthanation.

8. Procédé (20) de production de méthane selon l'une des revendications 1 à 7, dans lequel l'unité de méthanation est alimenté par un flux de gaz comportant un mélange stœchiométrique de CO₂ et de H₂.

9. Dispositif d'adaptation d'une unité de production de méthane à partir de biogaz pour fournir l'intrant dioxyde de carbone nécessaire au fonctionnement d'une installation de type power-to-gas, comportant :
- un moyen de fourniture au réacteur de méthanation d'un flux de dioxyde de carbone issu au moins en partie d'un moyen de séparation du dioxyde de carbone d'un flux comportant du biogaz produit par un digesteur,
- un réacteur de méthanation configuré pour produire un gaz de synthèse au moins à partir du flux de dioxyde de carbone fourni au réacteur et d'un flux riche en dihydrogène,
- un séparateur du gaz de synthèse en une fraction riche en dihydrogène d'une part et une fraction riche en méthane et en dioxyde de carbone d'autre part,
- une conduite de recirculation de la fraction riche en dihydrogène pour fournir ladite fraction au réacteur de méthanation, et
- une conduite de raccordement pour fournir la fraction riche en méthane et en dioxyde de carbone au moyen de séparation du dioxyde de carbone.

10. Installation (10) de production de méthane, comportant:
- un digesteur (105) configuré pour produire du biogaz,
- un séparateur (185) d'un flux riche en dioxyde de carbone d'un flux composé au moins en partie d'un flux de biogaz issu du digesteur,
- un moyen de fourniture du flux riche en dioxyde de carbone à un réacteur de méthanation,
- une réacteur de méthanation (145) configuré pour produire un gaz de synthèse au moins à partir du flux de dioxyde de carbone fourni au réacteur et d'un flux riche en dihydrogène,
- un séparateur (155) du gaz de synthèse en une fraction riche en dihydrogène d'une part et une fraction riche en méthane et en dioxyde de carbone d'autre part, et
- une conduite (157) de recirculation de la fraction riche en dihydrogène pour fournir le réacteur de méthanation (145) en dihydrogène.

11. Installation (10) pour la production de méthane selon la revendication 10, qui comporte un mixeur (180) configuré pour mélanger le biogaz avec la fraction riche en méthane et en dioxyde de carbone issu de la méthanation, ledit mixeur (180) étant placé en amont du séparateur (190) configuré pour séparer le mélange obtenu en une fraction riche en méthane et une fraction riche en dioxyde de carbone.

12. Installation (10) pour la production de méthane selon l'une des revendications 10 ou 11, qui comporte un électrolyseur (130) pour la production d'hydrogène pour fournir le réacteur de méthanation (145).

13. Installation (10) pour la production de méthane selon l'une des revendications 10 à 12, qui comporte une unité de désulfuration (110) du biogaz, une unité de déshydratation (115) du biogaz et une unité de déshydratation (150) du gaz de synthèse.

14. Installation (10) pour la production de méthane selon l'une des revendications 10 à 13, qui comporte un mixeur (135) configuré pour mélanger la fraction riche en dihydrogène acheminée depuis le séparateur d'hydrogène (155) avec le dihydrogène produit par l'électrolyseur (130) en vue d'alimenter le réacteur de méthanation (145).

## Patentansprüche

1. Verfahren (20) zur Produktion von Methan, umfassend:
- einen Schritt (205) zur Produktion von Biogas durch einen Faulbehälter,
- einen Schritt (290) zum Trennen eines Kohlendioxid-Stroms von einem Strom, der wenigstens zum Teil von einem Biogasstrom gespeist wird, der aus dem Schritt zur Produktion von Biogas stammt,
- einen Schritt (222) zur Lieferung des Kohlendioxid-Stroms an einen Methanisierungsreaktor,
- einen Schritt (245) zur Produktion von Synthesegas durch einen Methanisierungsreaktor wenigstens ausgehend von dem Kohlendioxid-Strom, der dem Reaktor geliefert wird, und von einem an Dihydrogen reichen Strom,
- einen Schritt (255) zum Trennen des Synthesegases in einen an Dihydrogen reichen Anteil einerseits und einen an Methan und an Kohlendioxid reichen Anteil andererseits und
einen Schritt (257) zum Zurückführen des an Dihydrogen reichen Anteils, um den genannten Anteil dem Methanisierungsreaktor zu liefern.

2. Verfahren (20) zur Produktion von Methan gemäß Anspruch 1, das umfasst:
- einen Schritt (280) zum Mischen des Biogases mit dem an Methan und an Kohlendioxid reichen, aus der Methanisierung stammenden Anteil,
- einen Schritt (290) zum Trennen der Mischung, die aus einem an Methan reichen Anteil und einem an Kohlendioxid reichen Anteil erhalten wird.

3. Verfahren (20) zur Produktion von Methan gemäß Anspruch 2, das dem Schritt (280) zum Mischen vorgeschaltet umfasst:
- einen Schritt (210) zur Entschwefelung des vom Faulbehälter produzierten Biogases und
- einen Schritt (215) zum Entwässern des vom Faulbehälter produzierten Biogases.

4. Verfahren (30) zur Produktion von Methan gemäß einem der Ansprüche 1 bis 3, bei dem der an Methan reiche Anteil einen Wasserstoffgehalt gemäß den Spezifikationen der Erdgasnetze aufweist.

5. Verfahren (20) zur Produktion von Methan gemäß einem der Ansprüche 1 bis 4, das umfasst:
- einen Schritt (230) zur Produktion von Dihydrogen per Elektrolyse und
- einen Schritt (243) zum Liefern des produzierten Dihydrogens an den Methanisierungsreaktor.

6. Verfahren (20) zur Produktion von Methan gemäß einem der Ansprüche 1 bis 5, das einen Schritt (235) zum Mischen des per Elektrolyse produzierten Dihydrogens mit dem an Dihydrogen reichen Anteil, der aus dem Schritt (255) zum Trennen des Synthesegases stammt, umfasst.

7. Verfahren (20) zur Produktion von Methan gemäß einem der Ansprüche 1 bis 6, das dem Schritt (255) zum Trennen des Gases vorgeschaltet einen Schritt (250) zum Entwässern des von dem Methanisierungsreaktor produzierten Synthesegases umfasst.

8. Verfahren (20) zur Produktion von Methan gemäß einem der Ansprüche 1 bis 7, bei dem die Methanisierungseinheit durch einen Gasstrom versorgt wird, der eine stöchiometrische Mischung aus CO₂ und H₂. umfasst.

9. Anpassungsvorrichtung einer Produktionseinheit von Methan ausgehend von Biogas, um den Kohlendioxid-Einsatz zu liefern, der für den Betrieb einer Anlage vom Typ Power-to-Gas notwendig ist, umfassend:
- ein Mittel zum Liefern eines wenigstens zum Teil aus einem Mittel zum Trennen des Kohlendioxids eines von einem Faulbehälter produzierten Biogas umfassenden Stroms stammenden Kohlendioxid-Stroms an den Methanisierungsreaktor,
- einen Methanisierungsreaktor, der zum Produzieren eines Synthesegases wenigstens ausgehend von dem Kohlendioxid-Strom, der dem Reaktor geliefert wird, und eines an Dihydrogen reichen Stroms, ausgestaltet ist,
- einen Abscheider des Synthesegases in einen an Dihydrogen reichen Anteil einerseits und einen an Methan und Kohlendioxid reichen Anteil andererseits,
- eine Rückführleitung des an Dihydrogen reichen Anteils zum Liefern des genannten Anteils an den Methanisierungsreaktor und
- eine Anschlussleitung zum Liefern des an Methan und an Kohlendioxid reichen Anteils an das Trennmittel des Kohlendioxids.

10. Anlage (10) zur Produktion von Methan, umfassend:
- einen Faulbehälter (105) zur Produktion von Biogas,
- einen Abscheider (185) eines an Kohlendioxid reichen Stroms von einem Strom, der wenigstens zum Teil aus einem Biogasstrom zusammengesetzt ist, das aus dem Faulbehälter stammt,
- ein Liefermittel des an Kohlendioxid reichen Stroms an einen Methanisierungsreaktor,
- einen Methanisierungsreaktor (145), der zum Produzieren eines Synthesegases wenigstens teilweise ausgehend von dem Kohlendioxid-Strom, der dem Reaktor geliefert wird, und einem an Dihydrogen reichen Strom ausgestaltet ist,
- einen Abscheider (155) des Synthesegases in einen an Dihydrogen reichen Anteil einerseits und einen an Methan und an Kohlendioxid reichen Anteil andererseits und
- eine Rückführleitung (257) des an Dihydrogen reichen Anteils, um den Methanisierungsreaktor (145) mit Dihydrogen zu beliefern.

11. Anlage (10) für die Methanproduktion gemäß Anspruch 10, die einen Mischer (180) umfasst, der zum Mischen des Biogases mit dem an Methan und an aus der Methanisierung stammenden Kohlendioxid reich ist, wobei der genannte Mischer (180) dem Abscheider (190) vorgeschaltet ist, der zum Trennen der Mischung ausgestaltet ist, die in einem an Methan reichen Anteil und einem an Kohlendioxid reichen Anteil erhalten ist.

12. Anlage (10) für die Methanproduktion gemäß einem der Ansprüche 10 oder 11, die einen Elektrolyseur (130) für die Wasserstoffproduktion zum Beliefern des Methanisierungsreaktors (145) umfasst.

13. Anlage (10) für die Methanproduktion gemäß einem der Ansprüche 10 bis 12, die eine Entschwefelungseinheit (110) des Biogases, eine Enwässerungseinheit (115) des Biogases und eine Entwässerungseinheit (150) des Synthesegases umfasst.

14. Anlage (10) für die Methanproduktion gemäß einem der Ansprüche 10 bis 13, die einen Mischer (135) umfasst, der zum Mischen des an Dihydrogen reichen Anteils, der vom Wasserstoffabscheider (155) dem Dihydrogen zugeführt ist, das vom Elektrolyseur (130) zur Versorgung des Methanisierungsreaktors (145) produziert ist, ausgestaltet ist.

## Claims

1. Methane production method (20), comprising:
- a step (205) of producing biogas by a digester;
- a step (290) of separating a carbon dioxide flow from a flow supplied at least in part by the flow of biogas coming from the biogas production step;
- a step (222) of supplying the carbon dioxide flow to a methanation reactor;
- a step (245) of producing synthetic gas by a methanation reactor from at least the carbon dioxide flow supplied to the reactor and a flow rich in hydrogen;
- a step (255) of separating the synthetic gas into, firstly, a fraction rich in hydrogen and, secondly, a fraction rich in methane and carbon dioxide; and
- a step (257) of recirculating the fraction rich in hydrogen to supply said fraction to the methanation reactor.

2. Methane production method (20) according to claim 1, which comprises:
- a step (280) of mixing biogas with the fraction rich in methane and carbon dioxide coming from methanation;
- a step (290) of separating the mixture obtained into a fraction rich in methane and a fraction rich in carbon dioxide.

3. Methane production method (20) according to claim 2, which comprises, before the mixing step (280):
- a step (210) of desulfurizing the biogas produced by the digester; and
- a step (215) of dehydrating the biogas produced by the digester.

4. Methane production method (20) according to one of claims 1 to 3, wherein the fraction rich in methane has a hydrogen content that complies with the specifications of the natural gas networks.

5. Methane production method (20) according to one of claims 1 to 4, which comprises:
- a step (230) of producing hydrogen by electrolysis; and
- a step (243) of supplying the hydrogen produced to the methanation reactor.

6. Methane production method (20) according to one of claims 1 to 5, which comprises a step (235) of mixing the hydrogen produced by electrolysis with the fraction rich in hydrogen coming from the synthetic gas separation step (255).

7. Methane production method (20) according to one of claims 1 to 6, which comprises, before the gas separation step (255), a step (250) of dehydrating synthetic gas produced to the methanation reactor.

8. Methane production method (20) according to one of claims 1 to 7, wherein the methanation unit is supplied by a gas flow comprising a stoichiometric mixture of CO₂ and H₂.

9. Adjustment device for a methane-from-biogas production unit to supply the carbon dioxide input required for a power-to-gas installation to operate, said device comprising:
- a means for supplying the methanation reactor with a carbon dioxide flow coming at least in part from a means for separating carbon dioxide from a flow comprising biogas produced by a digester;
- a methanation reactor configured to produce a synthetic gas from at least the carbon dioxide flow supplied to the reactor and a flow rich in hydrogen;
- a separator for separating the synthetic gas into, firstly, a fraction rich in hydrogen and, secondly, a fraction rich in methane and carbon dioxide;
- a recirculation line for recirculating the fraction rich in hydrogen to supply said fraction to the methanation reactor; and
- a connecting line to supply the fraction rich in methane and carbon dioxide to the carbon dioxide separation means.

10. Methane production installation (10), comprising:
- a digester (105) configured to produce biogas;
- a separator (185) for separating a flow rich in carbon dioxide from a flow comprised at least in part of a flow of biogas coming from the digester;
- a means for supplying the flow rich in carbon dioxide to a methanation reactor;
- a methanation reactor (145) configured to produce a synthetic gas from at least the carbon dioxide flow supplied to the reactor and a flow rich in hydrogen;
- a separator (155) for separating the synthetic gas into, firstly, a fraction rich in hydrogen and, secondly, a fraction rich in methane and carbon dioxide; and
- a recirculation line (157) for recirculating the fraction rich in hydrogen to supply the methanation reactor (145) with hydrogen.

11. Methane production installation (10) according to claim 10, which comprises a mixer (180) configured to mix the biogas with the fraction rich in methane and carbon dioxide coming from methanation, said mixer (180) being placed upstream of the separator (190) configured to separate the mixture obtained into a fraction rich in methane and a fraction rich in carbon dioxide.

12. Methane production installation (10) according to one of claims 10 or 11, which comprises an electrolyzer (130) for producing hydrogen to supply the methanation reactor (145).

13. Methane production installation (10) according to one of claims 10 to 12, which comprises a desulfurization unit (110) for desulfurizing biogas, a dehydration unit (115) for dehydrating biogas, and a dehydration unit (150) for dehydrating synthetic gas.

14. Methane production installation (10) according to one of claims 10 to 13, which comprises a mixer (135) configured to mix the fraction rich in hydrogen transported from the hydrogen separator (155) with the hydrogen produced by the electrolyzer (130) in order to supply the methanation reactor (145).
